# EUROPEAN PATENT SPECIFICATION

(11) **EP 0 824 674 B1**
(45) Date of publication and mention of the grant of the patent: **09.08.2000**
(21) Application number: 96915440.0
(22) Date of filing: 01.05.1996
(51) Int. Cl.: G01L 7/00, G01L 7/02, G01F 1/38

(54) **FLUID FLOW INDICATING APPARATUS**
DURCHFLUSSMESSGERÄT
APPAREIL INDICATEUR D'ECOULEMENT DE FLUIDE

(30) Priority: 01.05.1995 US 432220
(43) Date of publication of application: 25.02.1998
(73) Proprietor: SCIENCE INCORPORATED, Bloomington, MN 55437 (US)
(72) Inventor: KRIESEL, Marshall S., St. Paul, MN 55104 (US); ARNOLD, Steven M., Minnetonka, MN 55391 (US); GARRISON, James, Minneapolis, MN 55426 (US); KAZEMZADEH, Farhad, Bloomington, MN 55431 (US)
(74) Representative: Sutto, Luca, Ing.
(86) International application number: US9606099
(87) International publication number: WO9635106

(56) References cited:
- EP-A- 0 080 781
- US-A- 3 675 722
- US-A- 3 703 879
- US-A- 4 020 784
- US-A- 4 343 188
- US-A- 4 784 648

## Description

### BACKGROUND OF THE INVENTION

### 1. Field of the Invention

The present invention relates generally to devices for indicating fluid flow through fluid conduits. More particularly, the invention concerns a mechanical fluid flow indicator for providing a clear visual indication of fluid flow through one or more fluid conduits with which the indicator is associated.

### 2. Description of the prior art

The prior art is replete with devices for detecting and measuring fluid flow through fluid conduits. The devices range from simple pressure gages to highly complex mechanical and electrical detectors and flow meters.

Fluid flow indicating devices find application in numerous fields including chemical, industrial and medical fields. In the medical field a number of diverse applications exist for fluid delivery systems that incorporate a means for indicating the status of fluid flow to the patient. For example, traditional gravity feed intravenous delivery systems often incorporate a flow indicator that is positioned in the fluid delivery line that connects the fluid reservoir of the device to the patient. As exemplified by U. S. Patent No. 5,267,980 issued to Dirr, Jr. et al, an indicator of this type operates by passing a beam of electromagnetic energy from a light-emitting source through the drip chamber itself The passage of a drop of fluid through the drip chamber interrupts the light beam. which, in turn is registered by a light detector situated opposite the light emitting source. In addition to optical alignment, this type of system requires an external source of energy for light emitter and assembly of the system before it can be used.

The prior art also teaches the use of electronic sensors as detectors for pole mounted pumps used for intravenous administration of drugs to a patient, usually when very accurate delivery regimens or delivery of multiple fluids are required. One such application is disclosed in U. S. Patent No. 5,039,279, issued to Natwick et al which provides sensors to detect whether the medication is reaching the patient at the desired rate. In the Natwick et al device fail safe mechanisms work in conjunction with sensors which detect excessive flow rates and air bubbles in the line. Pole mounted pumps such as these, provide accurate systems for delivery, but are typically expensive to use and do not allow the patient to be ambulatory as they require an external power source.

A fluid flow indicator such as that described in U. S. No. 4,187,847 issued to Loeser embodies still another type of flow meter disposed in the fluid flow path of an airless fluid delivery apparatus. In this device, the flow indicator comprises a pin wheel or rotor comprised of a plurality of colored fins connected to a hub which rotates with a chamber as fluid passes through during delivery. The color pattern produced by the velocity of rotation of the rotor provides a visual indicator of the flow rate during operation of the fluid delivery system.

Yet another example of a prior art fluid flow detection system can be found in U. S. Patent No. 4,784,648, issued to Singh et al which describes an in-line flow restrictor used in conjunction with a source of pressurized infusate. Attached to and in fluid communication with the flow restrictor on both the upstream and downstream ends are annuli, which are inflatable by infusate pressure. In operation of the Singh et al device, the upstream annulus is inflated by the delivery pressure generated by the source, while the downstream annulus inflates only when pressure builds due to infiltration of the infusion site. When inflated, the annuli provide a visual indication through a jacket constructed from frosted material or alternatively by contacting a membrane switch.

U. S. Patent 3,895,631 issued to Buckles et al describes a portable, liquid infusion device which embodies still a different type of fluid flow indicator system. In the Buckles et al device, a viewing aperture and an open slot marked with a volume scale are provided on the device container cover. A visual signal appears in the viewing aperture that indicates to the patient when infusion has ceased due to a blockage present in the fluid flow path. Fluid pressure that would build up in the system should the needle or catheter become blocked causes the activation of a rotating arm that positions the signal in the view aperture on the device container. The volume scale in cooperation with an indicator arm attached to the fluid reservoir provide a means for indicating to the patient the volume of fluid remaining in the reservoir and when the reservoir is empty.

It is also known from document EP-A-0080781 a vacuum cleaner having a pressure sensitive device for indicating the strength of suction. The device comprises a membrane separating a housing in two spaces each communicating with different zones of the sucked flow. The diaphragm is displaced during working of the vacuum cleaner proportionally to the suction strength and moves a leaf spring which carries a terminal indicator or pin which is visible through a window. Therefore, the indicator may only detect and show the presence of more or less strength in the suction conduit.

Moreover, document US-A-3,675,722 shows a pressure indicator comprising a diaphragm or disc having a domed portion movable from a normal convex position to a high pressure concave position. A window is associated and complementary to said domed portion. The window is substantially frosted except for one portion which is transparent. When the diaphragm is in tight engagement with the frosted surface of the window, this latter presents a uniform appearance. When the diaphragm is disengaged from the window, the surface of the diaphragm becomes visible through the transparent areas of the window. This latter may present transparent areas configurated to indicate a word, for example "empty". A sheet formed member may be adhered to an outermost side surface of the diaphragm and moves with the domed portion. The flexible sheet formed member may contain indentations forming, for example, the word "full" which becomes visible when the diaphragm is urged against the window.

The prior art devices, while generally operating in a reasonably satisfactory manner, are often quite complex, difficult to maintain frequently require external power sources and sometimes are unreliable in long-term operation. It is these and other drawbacks of the prior art that the present invention seeks to overcome by providing a very simple, easily interpreted, and highly reliable mechanical apparatus for visually indicating fluid flow through a system or apparatus.

By way of summary, the apparatus of the invention for visually indicating fluid flow through a fluid system or apparatus comprises a pair of thin, indicia bearing films disposed in an overlaying relationship. These films are shifted relative to each other by movement of mechanical actuators which are deflected solely by the pressure of the fluid within the fluid conduits of the system of the appartus.

In the preferred form of the invention, the two films are stacked in close proximity and are constructed from a substantially transparent flexible material such as mylar. One surface of the inferior film is printed with a plurality of integrated symbols depicting various fluid flow conditions. The superior film functions as a mask over the inferior film and is printed with a pattern of diagonally extending, alternating clear and opaque stripes. The print ratio of the superior film permits viewing at any one time of only one of the symbols printed on the inferior film.

The inferior and superior films are attached at opposite ends to a support platform in a manner such that the patterned portions are in register and the nonpatterned portions cover actuator openings provided near each end of the support platform. Each film is able to move in opposing directions parallel to the film plane with its range of motion limited to one axis in the film plane. When the films move, the visible symbol pattern being viewed changes due to the transverse displacement of the printed symbols relative to the stripes on the superior film.

The mechanical actuators of the device are deflected from their initial, at-rest, generally planar position whenever there is a sufficient amount of fluid pressure present within the fluid conduit with which the particular actuator is associated. The actuator is deflected outwardly by the pressure of the fluid with sufficient force to urge the nonpatterned portion of the indicator film into an expansion space provided in a backing plate. As a portion of the film moves into the expansion space, the printed portion of the film is transversely displaced a specific predetermined distance. The displacement of the film realigns the printed symbol patterns on the inferior film with the mask pattern on the superior film causing a change in the symbols being viewable by the observer.

It is a primary object of the present invention to provide an apparatus for visually indicating the existence of fluid pressure within one or more fluid carrying conduits, which is of simple construction, is highly reliable in operation and is easily interpreted.

Another object of the invention is to provide an apparatus of the aforementioned character which in no way impedes the flow of fluid through the fluid conduits and is actuated solely by the fluid pressure of the fluid within the fluid conduits.

Another object of the invention is to provide an apparatus as described in the preceding paragraphs which distinguishes between and provides a clear indication of various conditions of fluid flow through a given fluid system or apparatus, such as normal flow, absence of fluid pressure within all or a portion of the system and abnormal or interrupted flow due to a blockage within the system.

Still another object of the invention is to provide a fluid flow indicator of the class described which is ideally suited for use in connection with medical infusion devices of the character having a fluid reservoir defined by a distendable membrane super-imposed over a base, the distendable membrane being distendable by the fluid to be infused into the patient and having a tendency to return to a less distended configuration in order to urge fluid flow through a fluid conduit associated with the flow indicator.

Another object of the invention is to provide a fluid flow indicator as described in the preceding paragraphs in which normal flow of fluid from the reservoir of the infusion device is clearly indicated by indicia-carrying thin films disposed in an overlaying configuration which films are movable relative to each other by mechanical actuators operated solely by fluid under pressure flowing from the reservoir. Another object of the invention is to provide an apparatus of the character described which embodies few parts, requires virtually no maintenance and can be easily and inexpensively manufactured.

### BRIEF DESCRIPTION OF THE DRAWINGS

Figure 1 is a generally perspective, exploded view of one form of the fluid flow indicator apparatus of the invention.

Figure 2 is a cross-sectional view of the apparatus of Figure 1 as it appears in an assembled state.

Figure 3 is a front view of a pair of indicia bearing films of the apparatus which are adapted to be disposed in an overlaying configuration.

Figure 4 is a generally diagrammatic front view showing the symbol that is viewable when the films are in their initial starting position.

Figure 5 is a generally diagrammatic top view showing the pair of indicia bearing films of the apparatus in a shifted position indicating fluid flow through the device.

Figure 6 is a generally diagrammatic front view showing the symbol that is viewable when the films are in the position shown in Figure 5.

Figure 7 is a generally diagrammatic top view showing the pair of indicia bearing films of the apparatus in shifted position indicating an absence of fluid flow through the outlet port of the device.

Figure 8 is a generally diagrammatic frontview showing the symbol that is viewable when the films are in the position shown in Figure 7.

Figure 9 is a generally perspective top view of an alternate form of the apparatus of the invention showing the flow indicator embodied in a medical infusion device of the character used to infuse medicinal fluids into a patient.

Figure 10 is a generally perspective, bottom view of the apparatus shown in Figure 9.

Figure 11A is a generally perspective, exploded view of the downstream portion of the apparatus of Figures 9 and 10 showing the flow indicator and a portion of the flow control means.

Figure 11B is a generally perspective, exploded view of the remainder of the flow control means along with the reservoir subassembly portion of the apparatus shown in Figures 9 and 10.

Figure 11C is a generally perspective, fragmentary view of a portion of the distendable membrane assembly of the apparatus.

Figure 12 is a top plan view of the apparatus, partly broken away to show internal construction.

Figure 13 is a cross-sectional view taken along lines 13-13 of Figure 12.

Figure 14 is a cross-sectional view taken along lines 14-14 of Figure 13.

Figure 15 is a cross-sectional view taken along lines 15-15 of Figure 13.

Figure 16 is a view of the apparatus taken along lines 16-16 of Figure 13.

Figure 17 is a cross-sectional view taken along lines 17-17 of Figure 16.

Figure 18 is a cross-sectional view taken along lines 18-18 of Figure 16.

Figure 19 is a cross-sectional view taken along lines 19-19 of Figure 16.

Figure 20 is a generally perspective, exploded view of the forward portion of the apparatus.

Figure 21 is a front view of the apparatus.

Figure 21A is a cross-sectional view taken along lines 21A-21A of Figure 21.

Figure 22 is a generally perspective view of the cover for the rate control apparatus of the invention.

Figure 23 is a generally perspective, front view of the substrate portion of the rate control apparatus.

Figure 24 is a generally perspective rear view of the substrate portion.

Figure 25 is a generally perspective view of the output port of the apparatus.

Figure 26 is a front view of the output port shown in Figure 18.

Figure 27 is a cross-sectional view taken along lines 27-27 of Figure 26.

Figure 28 is a bottom view of the luer valve fitting of the apparatus.

Figure 29 is a cross-sectional view taken along lines 29-29 of Figure 28.

Figure 30 is an enlarged front view of the indicia carrying thin films of the apparatus of the invention.

Figure 31 is a cross-sectional iew similar to Figure 21A showing the indicator means of the invention in its starting configuration.

Figure 32 is a fragmentary front view showing the symbol that is viewable by the user when the apparatus is in the configuration shown in Figure 31.

Figure 33 is a cross-sectional view similar to Figure 31, but showing the indicator means as it appears when fluid is flowing through the apparatus in a normal fashion.

Figure 34 is a fragmentary front view showing the symbol that is viewable by the user when the apparatus is in the configuration shown in Figure 33.

Figure 35 is a cross-sectional view similar to Figure 31, but showing the indicator means as it appears when there is a blockage downstream of the indicator means that prevents normal fluid flow.

Figure 36 is a fragmentary front view showing the symbol that is viewable by the user when the apparatus is in the configuration shown in Figure 35.

### DETAILED DESCRIPTION OF THE INVENTION

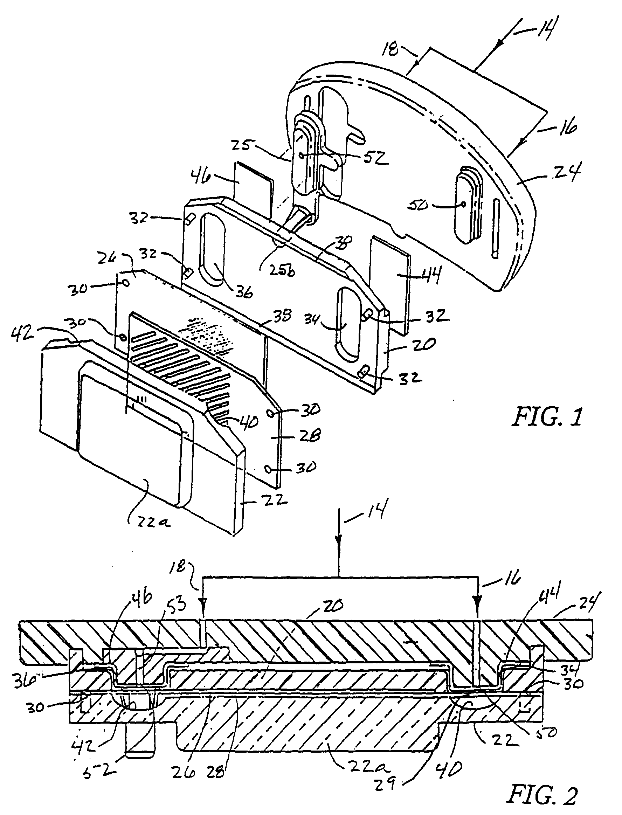

Referring to the drawings and particularly to Figures 1 and 2, one form of the fluid flow indicator means of the invention is there shown in fluid communication with a main fluid supply line 14 that branches into first and second feeder lines or conduits 16 and 18 respectively. The indicator means here comprises an indicator base or platform 20 which is connected to a support or lens plate 22 having a viewing lens portion 22a. Connected to a substrate 24 is an outlet port assembly 25 which has a fluid outlet port 25b.

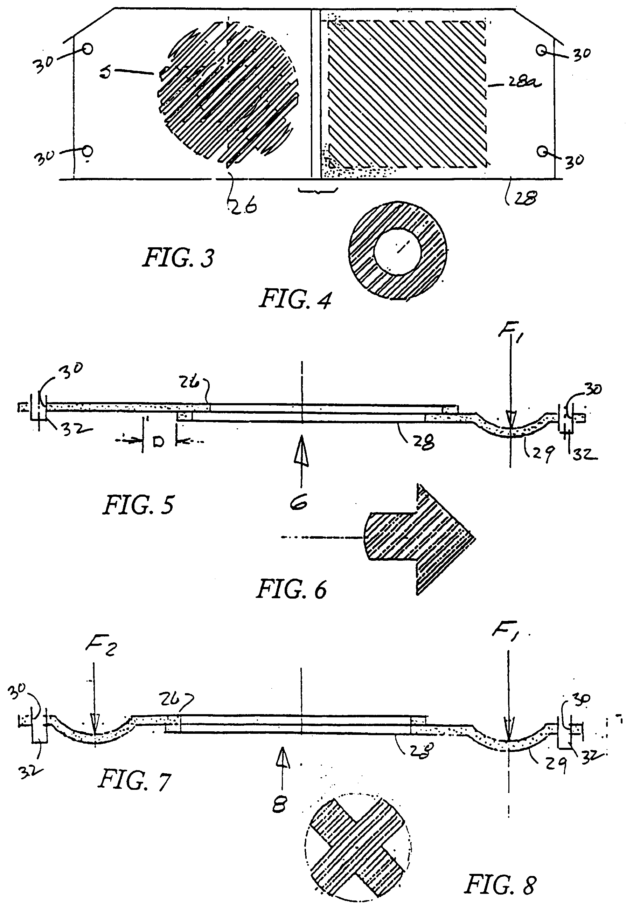

Disposed between platform 20 and plate 22 are first and second indicia-carrying means shown here as thin films 26 and 28. Films 26 and 28, which are in intimate contact, are preferably constructed from a substantially transparent, flexible material such as mylar. It is to be understood that the indicia-carrying means can take various forms and can comprise any structure having a surface that will display a selected indicia. As indicated in Figure 3, the downstream surface of the inferior or first film 26 is printed with three integrated symbols "S", namely, a blue circle, a green arrow, and a red X, each consisting of diagonal stripes of color printed in an alternating pattern as, for example, blue, green, red, blue, green red, and so on (see also Figures 4, 6, and 8). The superior, or second film 28 serves as a "mask" over the inferior film and is printed with a pattern of diagonal alternating clear and opaque stripes 28a that occur in a 1:2 ratio (Figure 3). The printed ratio of the superior "mask" allows only one colored symbol to appear at a time when viewed through a viewing lens portion 22a provided in plate 22.

The inferior and superior films are provided at their opposite ends with apertures 30 that receive retention pins 32 provided on platform 20 (Figure 1) which permit attachment of the film to platform 20 in a manner such that the patterned portions of the films are in index and the non-patterned portions of the films cover actuator slots 34 and 36 provided proximate each end of platform 20. With this construction, each thin film is able to move in opposing directions parallel to the film plane with its range of motion limited to one axis in the film plane by edge guides 38 provided on platform 20 (Figure 1). As the films move, the visible symbol pattern viewed through viewing lens 22a changes due to the transverse displacement of the patterns imprinted on the films (see Figures 4 through 8).

Referring to Figure 2, it is to be noted that support plate 22 is provided with transversely spaced, channel-like depressions 40 and 42 which index with slots 34 and 36 respectively when the components are assembled in the manner shown in Figure 2. Aligned with slots 34 and 36 are mechanical actuator means here provided as yieldably deformable mechanical actuators such as thin elastomeric elements 44 and 46. More particularly, first actuator element 44 aligns with slot 34 while the second actuator element 46 aligns with slot 36.

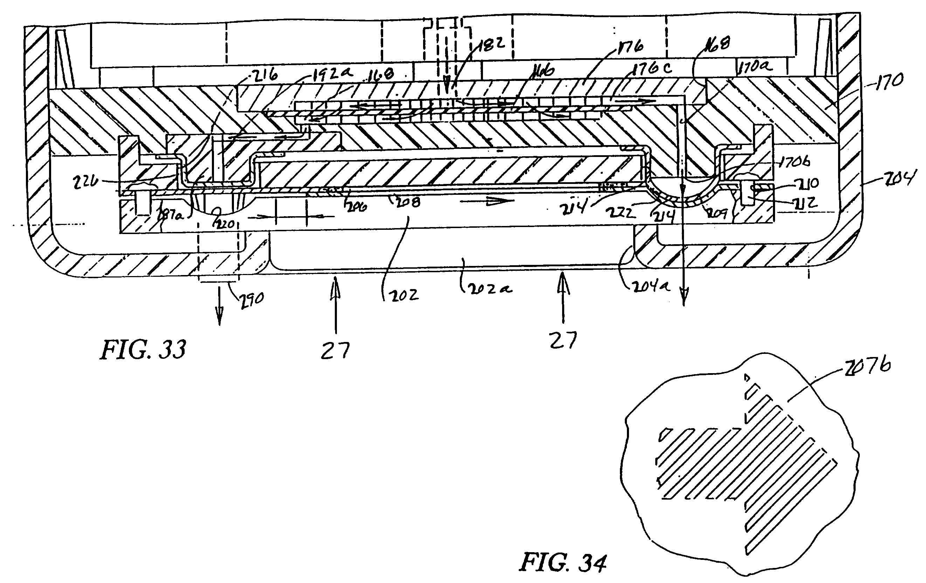

In the manner presently to be described, the mechanical actuator elements are deflected outwardly from their initial configuration whenever there is sufficient fluid pressure present within the feeder lines to cause their outward deflection as a result of fluid under pressure impinging on the actuator means via small apertures 50 and 52 provided in substrate 24. More particularly, during operation of the apparatus the first mechanical actuator element 44 is deflected by fluid pressure F-1 of fluid flowing through first conduit 16 and through aperture 50 provided in substrate 24. As depicted in Figures 5 and 33, as the first mechanical actuator means is deflected outwardly by fluid force F-1, the non-patterned portion 29 of indicator film 28 will be urged into expansion channel 40. As the film arches into channel 40, the printed portion of the film is transversely displaced a specific distance "D" (Figure 5). This film displacement re-aligns the printed symbol patterns on the inferior film 26 with the mask pattern on the superior film 28 and results in a change of the symbol (in this case the arrow shown in Figure 6) that is visible through the support plate viewing aperture 22a.

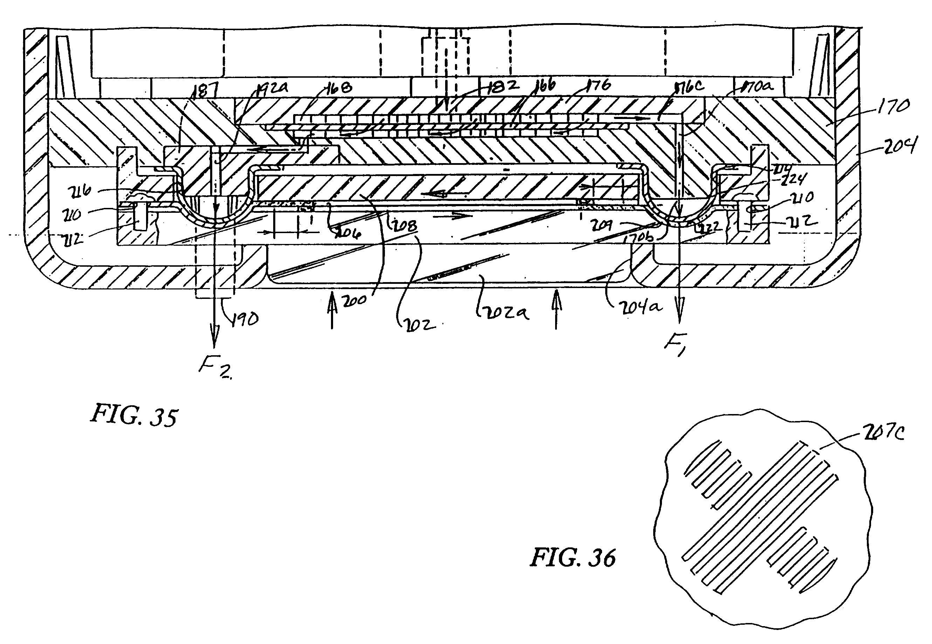

As can be understood by referring to Figures 7 and 35, both the first and second mechanical actuator elements 44 and 46 are deflected outwardly toward their respective extension channels when feeder lines 16 and 18 are pressurized by fluid forces F-1 and F-2. While first elastomeric element 44 will be deflected by line pressure F-1, second elastomeric element 46 is constructed so that a greater pressure F-2 is required to cause its deflection. In the present embodiment of the invention, this occurs when fluid is not flowing through outlet port 25b thereby causing pressure buildup within a passageway 53 leading to aperture 52 (Figure 2). When both mechanical actuators are deflected outwardly in the manner shown in Figure 7, both the superior and inferior films are displaced transversely to a second position revealing a second symbol, as for example, an X as viewed through the viewing aperture of the support plate (see Figures 8 and 36).

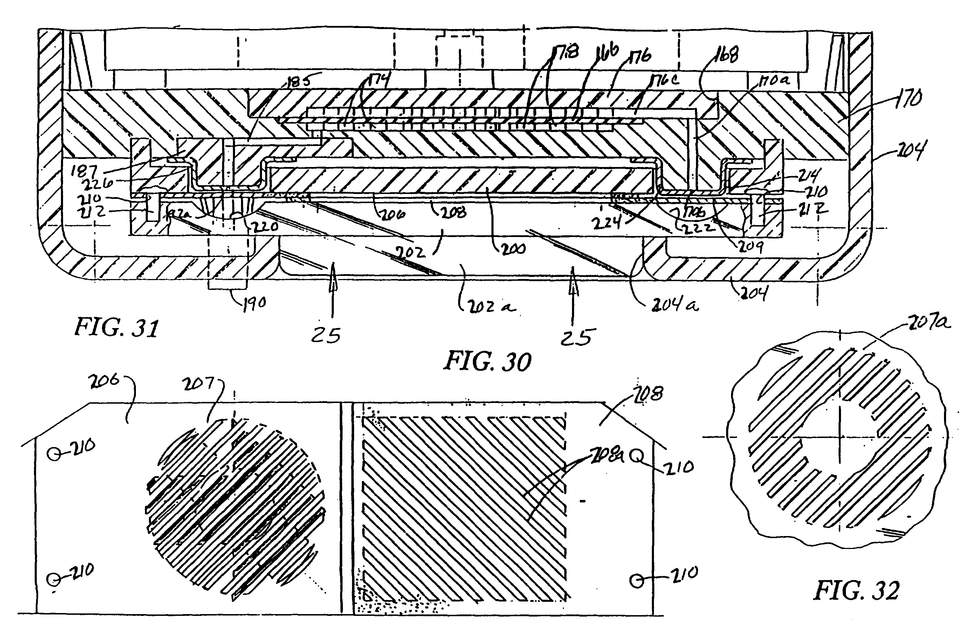

A third alignment of symbol patterns as indicated in Figures 3, 4, 31, and 32 is visible when the device is in an unfilled state or when no fluid is flowing through conduits 16 and 18. In this instance, there is no fluid pressure exerted on either of the first and second mechanical actuator elements and, therefore, the elements remain in a non-deflected configuration. Accordingly, the inferior and superior films are not transversely displaced and thus exhibit a third combination of patterns resulting in a third symbol as, for example, a circle being visible through the viewing aperture of the support plate (Figures 4 and 32). The actuator elements can be specially tailored to appropriately deflect under various pressure conditions thereby making the apparatus extremely versatile.

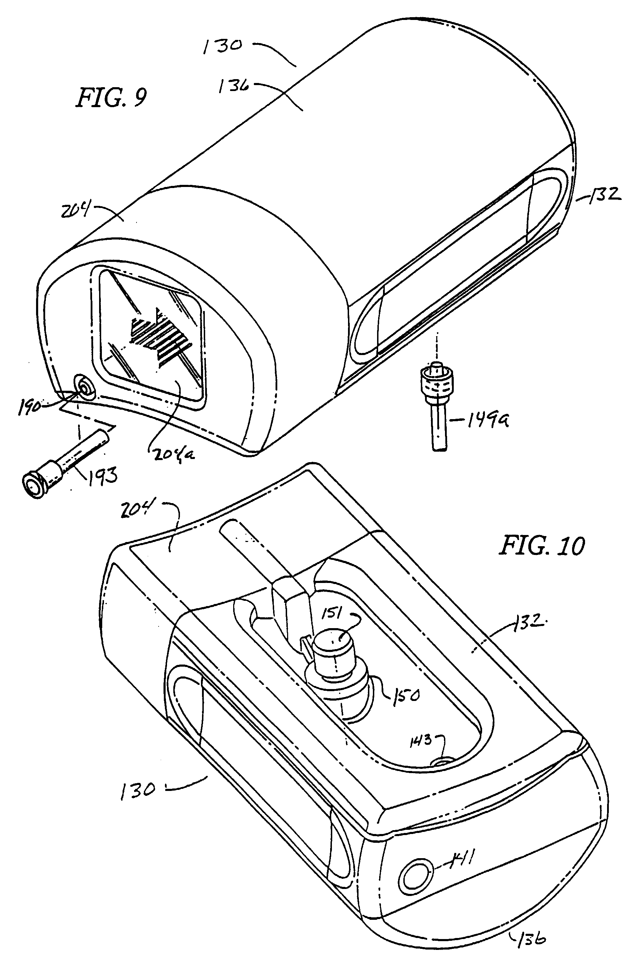

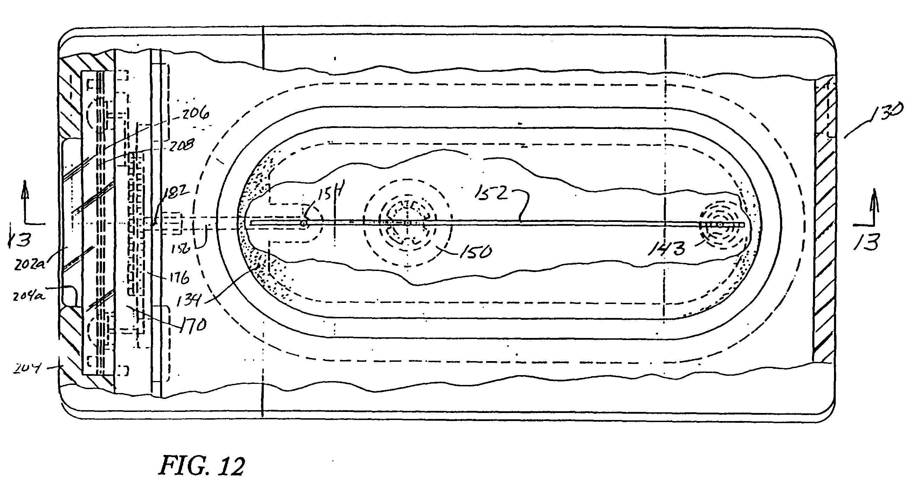

A particularly attractive use for the apparatus of the present invention is found in the medical field. More particularly, because of the simplicity, reliability and ease of use of the indicator devices of the invention, their use in combination with medical infusion devices provides an elegant means for providing a clearly interpreted visual display of the operating condition of the infusion device. Such a use is illustrated in Figures 9 through 36 wherein the indicator apparatus of the invention comprises one of the three major operating subsystems of a novel ambulatory medical infusion device 130 for delivering beneficial agents, such as medicaments to a patient.

Referring particularly to Figures 9 through 16, the apparatus there shown comprises three major cooperating subassembies namely, a reservoir subassembly, a flow rate control subassembly, and a flow indicator subassembly which embodies a form of indicator means somewhat similar to that shown in Figures 1 and 2. Each of the three subassemblies will be discussed in detail in the paragraphs which follow.

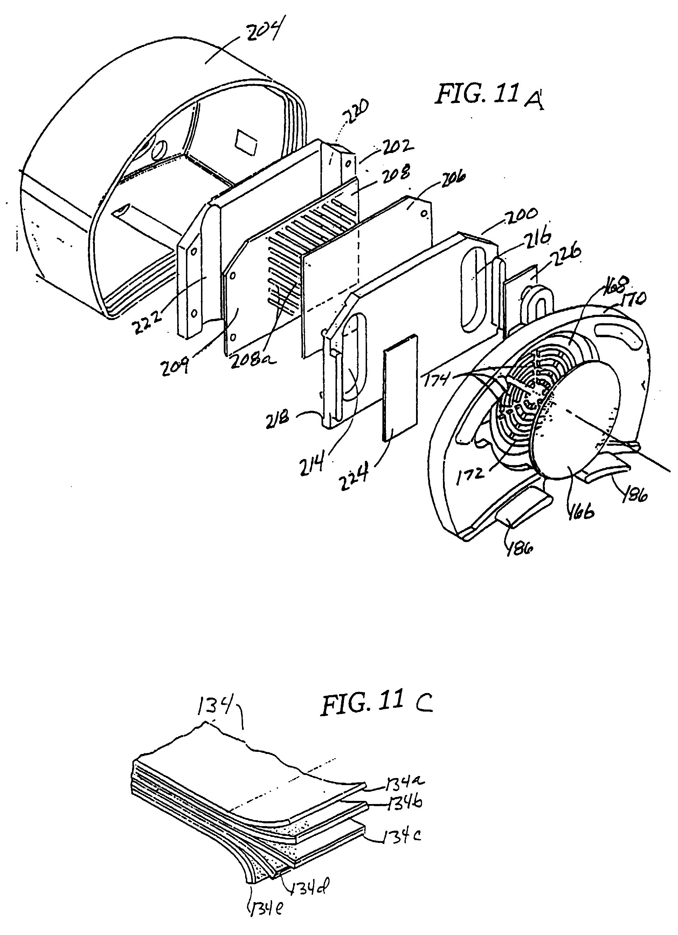

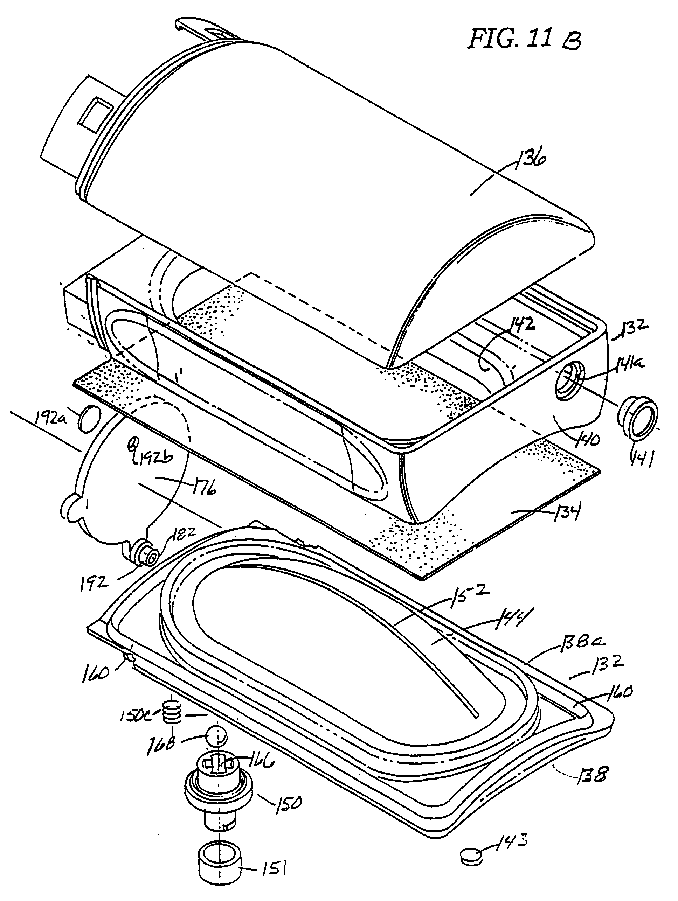

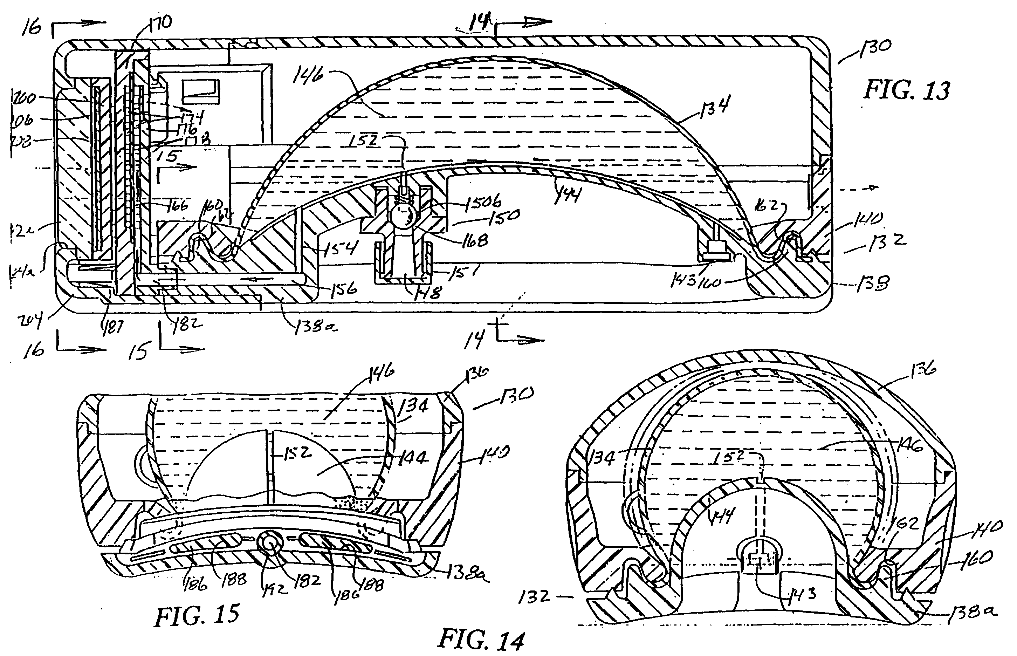

Considering first the reservoir subassembly shown in Figure 11B, this subassembly includes a base assembly 132, a stored energy source, or distendable membrane assembly 134, and a cover 136 for enclosing the stored energy source and the base assembly (see also Figures 13 and 14). The base assembly includes a ullage substrate 138 and a membrane capture housing 140 having a bottom opening 142 which receives the distendable membrane engaging element or protuberance 144.

Referring particularly to Figures 11B and 13, the ullage substrate 138 comprises, in addition to the distendable member engaging protuberance or ullage 144, filling means which enables the fluid reservoir 146 formed between protuberance 144 and distended membrane 134 to be filled. This filling means here includes a fluid inlet 148 provided in a luer valve fitting 150, the character of which will presently be described. Filling 150 is adapted to be interconnected with a filing conduit 149a (Figure 9). Protuberance 144 is provided with a longitudinally extending fluid passageway 152 (Figure 11B) which communicates with fluid passageways 154 and 156 provided in the base portion 138a of ullage substrate 138 (see also Figures 13 and 14).

Base portion 138a of ullage substrate 138 includes an upstanding tongue 160 which extends about the perimeter of the base portion and is closely receivable within a groove 162 formed in the base of membrane capture housing 140 (Figures 11B and 13). When the ullage substrate and the membrane capture housing are assembled in the manner shown in Figure 13, the periphery of distendable membrane assembly 134 will be securely clamped within groove 162 by tongue 160. After the parts are thus assembled, housing 140 is bonded to substrate 138 by any suitable means such as adhesive or sonic bonding. This done, cover 136 is mated with housing 140 in the manner shown in Figure 13 and bonded in place. Cover 136 is preferably constructed from a substantially transparent plastic material which is impermeable to fluids, including gases.

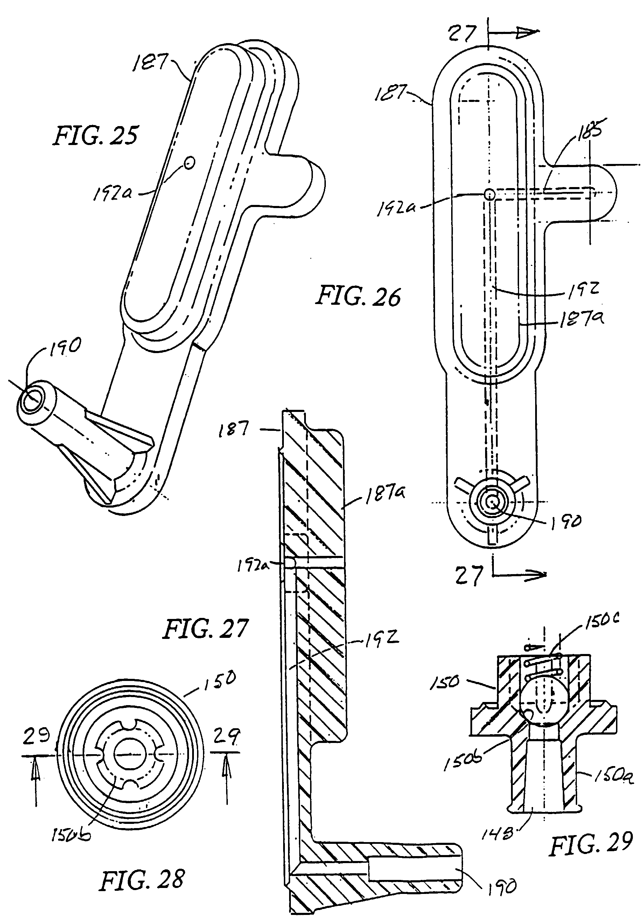

Filling of reservoir 146 is accomplished by introducing fluid under pressure into inlet passageway 148 and thence into reservoir 146 via filling conduit 149a and luer fitting 150. As the fluid under pressure flows into the reservoir, it will cause membrane assembly 134 to distend outwardly from protuberance 144 in the manner shown in Figure 13. Luer fitting 150 includes a skirt portion 150a, a valve seat 150b and a biasing spring 150c which biases a ball check valve 168 toward seat 150b (see also Figure 29). Ball 168 will lift from seat 150b against the urging of spring 150c during reservoir filling, but will sealably engage seat 150b after the reservoir has been filled. Inlet 148 is closed by a closure cap 151 prior to and following the filling step.

While the stored energy means can be in the form of a single prestressed or unstressed isotropic, elastomeric distendable membrane, it is here shown as a laminate assemblage made up of a plurality of initially generally planar distendable elements or films. Referring particularly to Figure 11C, the stored energy means can be seen to comprise a laminate assemblage made up of individual layers 134, 134a, 134b, 134c, and 134d. Assemblage 134, which is typically prestressed, cooperates with ullage substrate 138 to define a fluid chamber, or reservoir 146. By constructing the stored energy means from a composite of distinct elements or layers, the elastic characteristics of the stored energy means can be precisely tailored to meet end application requirements. As previously discussed, as the distendable membrane assemblage 134 is distended by the fluid pressure exerted by the fluid flowing into inlet 148, internal stresses are formed therein which continuously urge the assemblage toward engagement with protuberance 144 as the assemblage tends to return toward its original configuration. As assemblage 134 moves toward protuberance 144, fluid within reservoir 146 will be uniformly and controllably forced outwardly through longitudinally extending passageway 152 in protuberance 144 and then into passageways 154 and 156 of portion 138a of ullage substrate 138. Various types of hard plastic materials that can be used to construct the base assembly and the cover. Similarly, the membrane assemblage can be constructed from a wide variety of elastomers and similar materials of a character well known in the art.

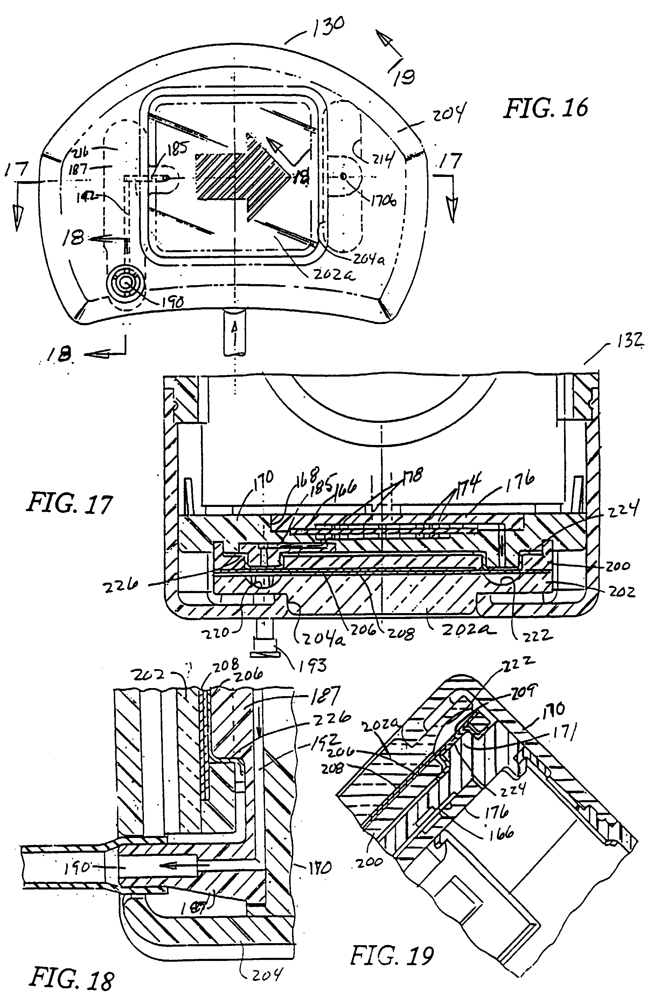

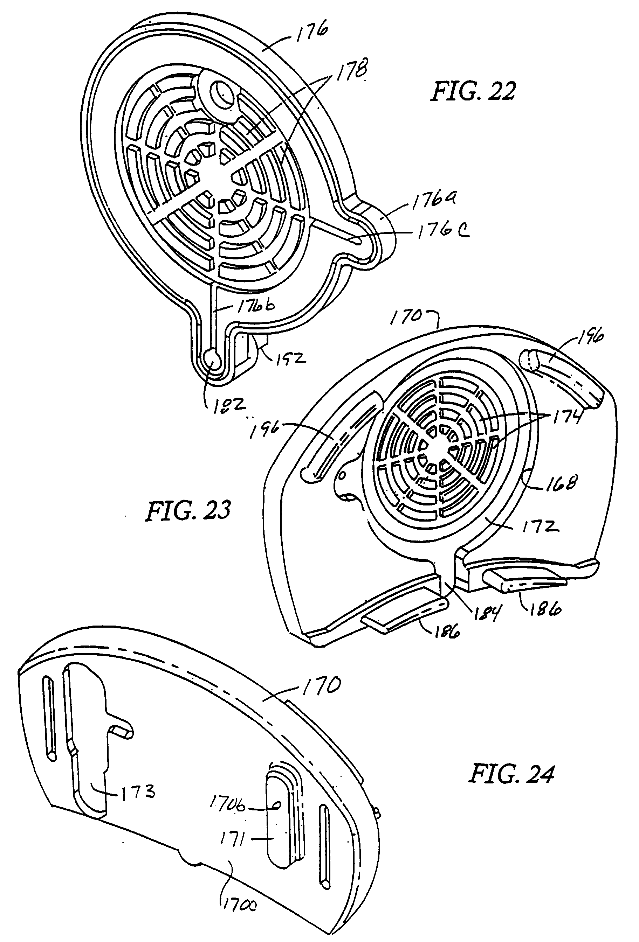

Turning next to a consideration of the flow rate control subassembly of the invention, this subsassembly includes novel flow control means which are disposed externally of reservoir 146 for controlling the rate of fluid flow of fluid from the device. In the embodiment of the invention shown in Figures 9 through 36, the flow control means comprises a rate control membrane 66 (Figure 11A) which is closely received within a circular recess 168 formed in support means shown here as a membrane support structure 170. The downstream wall 172 of recess 168 is provided with fluid distribution means comprising a multiplicity of circumferentially spaced, arcuate-shaped, manifolding stand-off elements 174 against which membrane 166 is held in engagement by a disc-like member 176 (Figure 13) which is receivable within recess 168 (see also Figure 22). As best seen by also referring to Figures 19 and 22, member 176 is provided with fluid collection means shown here as a multiplicity of circumferentially spaced, manifolding stand-offs 178 which engage membrane 166 when member 176 is in position within cavity 168. More particularly, as indicated in Figure 13, when member 176 is in place within cavity 168, the flow control membrane 166 is bonded at its circumference to member 170 and is securely positioned between stand-offs 174 and 178 which cooperate to define a multiplicity of concentric and radial extending fluid passageways, which function to direct fluid flow through the flow control means. Air within chamber 168 is vented via vent patch 192a and opening 192b (Figure 11B).

As best seen in Figures 11B and 22, member 176 includes a downwardly extending fluid inlet leg or segment 180 which is provided with a fluid passageway 182. Passageway 182 is adapted to communicate with chamber 168 when member 176 is mated with support structure 170. As shown in Figure 23, support structure 170 has a centrally disposed recess 184 that receives inlet segment 180.

Formed on either side of recess 184 are wing-like protuberances 186 that are received within spaced-apart, arcuate-shaped cavities 188 formed in the base portion 138a of ullage substrate 138. Also formed in substrate 138 is a socket 190 (Figure 13) which closely receives a tubular extension 192 formed as a part of inlet segment 180. Located proximate the upper edge of support structure 170 are spaced-apart capture grooves 196. which attach cover 136 to member 170.

As shown in Figures 13 and 17, when the flow control subassembly is mated with the reservoir assembly, fluid inlet passageway 182 of member 176 is placed in fluid communication with reservoir 146 via passageways 154 and 156. With this construction, when fluid is forced through fluid passageway 152 of protuberance 144 by the stored energy means, the fluid will flow into passageway 154, next into passageway 156 then into passage 182 of member 76, and finally into chamber 168 formed in member 170. As the fluid under pressure flows into the upstream portion of chamber 168 behind membrane 166, it will be distributed by stand-offs 178 so that it will uniformly flow through membrane 166 and toward the fluid outlet port of the flow control subassembly. As best seen in Figures 19 and 25, the outlet port comprises an assembly 187 which is receivable in a cavity 173 formed in the back of downstream wall 170a of the substrate 170. Assembly 187 includes a fluid outlet 190 and an internal chamber 192, the purpose of which will presently be described. During filling of chamber 192, air therewithin can be vented to atmosphere via vent patch 92a.

The flow control means of this form of the invention is shown as comprising a single layer of permeable material having the desired fluid flow characteristics. However, the flow control means can also comprise an assemblage of a plurality of layers of permeable materials, P-1, P-2, and P-3 of the character seen in Figure 31 of U. S. Patent No. 5,205,820 issued to the present inventors. These layers, which may be composites, thin films, or porous substrates, may be constructed of any one of the materials described in U. S. Patent No. 5,205,820 so that the fluid pressure flow characteristics of the assemblage can be precisely tailored for the particular medicinal or other fluid being dispensed. Reference should be made to U. S. Patent No. 5, 205,820 for a further description of the construction and operation of the flow control membrane and for a further discussion of the materials that can be used to construct the base, the cover and the stored energy means.

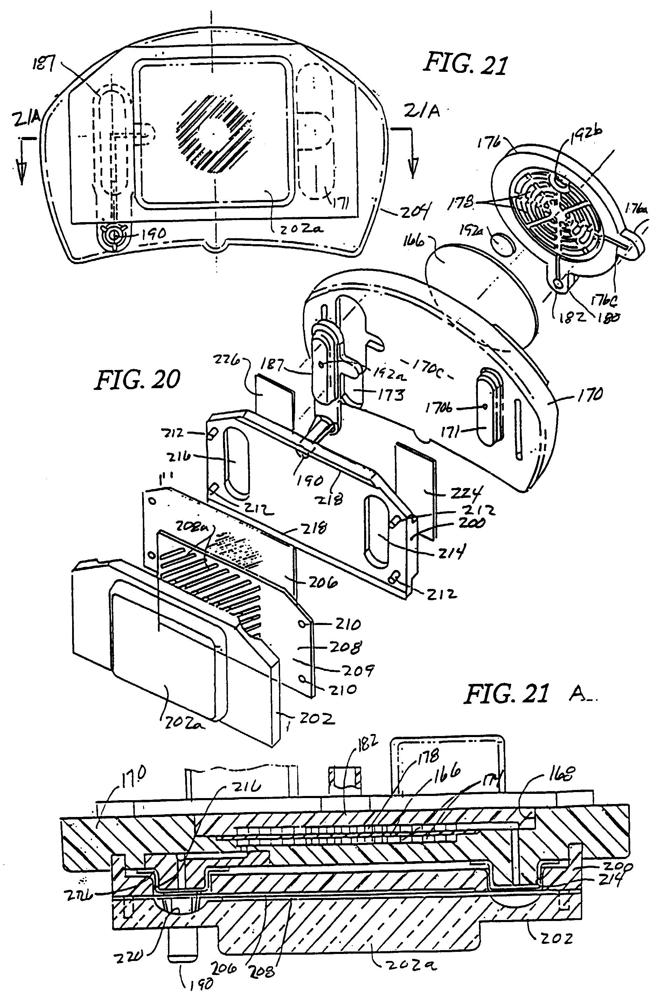

Considering now the flow indicator means of this embodiment of the invention, this novel means visually distinguishes among three conditions of operation, namely normal fluid flow, fluid flow blockage, and reservoir empty. Turning to Figure 11A, the flow indicator means here comprises an indicator base or platform 200, a support or lens plate 202, and a hollow housing 204 within which the platform and the support plate are mounted. As seen in Figure 21, plate 102 has a viewing lens 202a which indexes with an aperture 204a provided in housing 204.

Disposed between platform 200 and plate 202 are first and second indicia-carrying means shown here as thin films. These films, identified here as 206 and 208, are in intimate contact and are constructed from a substantially transparent, flexible material such as mylar. Once again, the indicia-carrying means can be any surface presenting member upon which indicia can be provided. The downstream surface of the inferior or first film 206 is printed with three integrated symbols 207 (Figure 30), namely, a blue circle 207a (Figure 32), a green arrow 207b (Figure 34), and a red X 207c (Figure 36), each consisting of diagonal stripes of color printed in an alternating pattern (blue, green, red, blue, green red, and so on (Figures 30 through 36)). The superior, or second film 208 serves as a "mask" over the inferior film 206 and is printed with a pattern of diagonal alternating clear and opaque strips 108a that occur in a 1:2 ratio. The printed ratio of the superior "mask" allows only one colored symbol to appear at a time when viewed through viewing lens 202a in plate 202. The inferior and superior films are provided at their opposite ends with apertures 210 which receive retention pins 212 provided on platform 200 (Figure 19) which permit attachment of the film to platform 212 in a manner such that the non-patterned portions of each film covers actuator slots 214 and 216 provided proximate each end of platform 200 with the patterned portions of both the superior and inferior films being maintained in index. With this construction, each thin film is able to move in opposing directions parallel to the film plane with its range of motion limited to one axis in the film plane by edge guides 218 provided on platform 200 (Figure 19). As the films move, the visible symbol pattern changes due to the transverse displacement of the patterns imprinted thereon.

Referring particularly to Figures 11A and 17, it can be seen that support plate 202 is provided with transversely spaced, channel-like depressions 220 and 222 which index with slots 214 and 216 respectively when the components are assembled in the manner shown in Figures 17 and 21. Aligned with the upstream (reservoir) side of slots 214 and 216 are mechanical actuator means here provided as mechanical actuators or elastomeric elements 224 and 226. More particularly the first actuator element 224 covers slot 214 and the second actuator element 226 covers slot 214.

In a manner presently to be described, the mechanical actuator means are deflected from their initial configuration whenever there is sufficient fluid pressure present within the fluid flow path to cause their outward deflection toward films 206 and 208. During operation the first mechanical actuator element 224 is deflected by fluid pressure of reservoir 146. More particularly, when there is sufficient fluid pressure in the fluid reservoir and fluid is being delivered by the stored energy means of the device, the first mechanical actuator means is deflected outwardly so as to urge the non-patterned portion 209 of indicator film 208 into expansion channel 222. As the film arches into channel 222, the printed portion of the film is transversely displaced a specific distance. This film displacement re-aligns the printed symbol patterns on the inferior film 206 with the mask pattern on the superior film 208 and results in a change of the symbol (in this case an arrow) see Figures 33 and 34 that is visible through the support plate viewing lens 202a.

As can be observed by referring to Figure 34, both the first and second mechanical actuator elements 224 and 226 are deflected outwardly toward their respective extension channels when the device is filled and primed but not in a state of delivery or when there is a build up of fluid pressure during delivery that is caused by blockage of the delivery line downstream from second mechanical actuator element 226. While element 224 can be deflected by normal line pressure element 226 is deflected only by pressure caused by a downstream blockage. When both mechanical actuators are deflected outwardly, both the superior and inferior films are displaced transversely to a second position revealing a second symbol, as for example, an X as viewed through the viewing aperture of the support plate (see Figure 36).

A third alignment of symbol patterns as shown in Figure 32is visible when the device is in an unfilled state or when the delivery line is open, the reservoir is empty and fluid delivery to the patient has been completed. In this case, there is no fluid pressure in the line on either the upstream or downstream side of the flow control means and thus both the first and second mechanical actuator elements are in a non-deflected position. In this condition, the inferior and superior films are not transversely displaced and thus exhibit a third combination of patterns resulting in a third symbol as. for example, a circle being visible through the viewing aperture of the support plate (see Figures 31 and 32).

In considering the method of operation of the device and the manner in which fluid flow through the device, reference should be made particularly to Figures 13, and 31 through 36. During the filling step, the fluid to be dispensed is introduced into reservoir 146 via a fluid inlet conduit 149a (Figure 9) which is connected to luer fitting 150. Fluid flowing into the fitting lifts check valve ball 168 against the urging of spring and causes the distendable membrane assembly to be displaced away from ullage protuberance 144 in the manner shown in Figure 13. Air within housing 140 and cover 136 will be suitably vented to atmosphere via a vent 141 which is receivable within a vent aperture 141a provided in housing 140 (Figure 11B). During the filling step, the gaseous component of the fluid is vented to atmosphere via a vent patch 143 provided in portion 138a of substrate 138 (Figures 11B and 13).

During the fluid dispensing step, the prestressed membrane assembly will tend to return toward a less distended configuration causing fluid within the chamber to flow outwardly of passageway 152 and into passageways 154 and 156. The fluid under pressure will next flow into passageway 182 of disc-shaped member 176. Turning particularly to Figures 17 and 22, it is to be observed that a portion of the fluid entering chamber 168 of member 170 from passageway 182 and upstream of membrane 166, can flow directly toward an ear-shaped extension 176a provided on member 176 via flow passageways 176b and 176c. From passageway 176c, the fluid will flow under pressure into passageway 170a formed in substrate 170 and toward passageway outlet 170b. Fluid flowing through outlet 170b will impinge directly upon flow indicator element 224 which sealably engages a protuberance 171 formed on substrate 170 causing element 224 to deform outwardly in a manner to force portion 209 of indicator film 208 to arch into expansion channel 222. This, in turn, will cause transverse displacement of indicator film 208 in the manner previously described.

Fluid flowing through passageway 182 of disc-shaped member 176 will also be distributed over the upstream face of the rate control membrane 166 by the fluid distribution means, or protuberances 178 and will pass through the membrane at a predetermined controlled rate. The fluid flowing through the rate control membrane will be collected by the fluid collection means or protuberance 174 and then will flow via passageway 185 into passageway 192 of outlet port assembly 187. The fluid will then flow outwardly of the device through fluid outlet 190 to which an infusion line 193 is connected (Figures 9, and 33). It is to be observed that a portion of the fluid flowing into outlet port assembly 187 is free to flow through passageway 192a provided in a protrubing portion 187a thereof. If there is a blockage which prevents free fluid flow outwardly of the device through outlet 190 and infusion line 193, fluid buildup pressure F-2 caused by the blockage will act upon indicator element 226 causing it to deflect outwardly. This outward deflection of element 226 will urge a portion of indicator film 206 into receiving channel 226 of the lens plate causing transverse movement of film 206 so as to reposition film 206 relative to film 208. Should fluid flow into chamber 192 cease, indicator element 226 will return to its at-rest position as will film 206. Similarly, if fluid flow from the reservoir ceases, film 208 will also return to its at rest position thereby once again causing the "O" symbol to be viewable through viewing lens 202a.

Having now described the invention in detail in accordance with the requirements of the patent statutes, those skilled in this art will have no difficulty in making changes and modifications in the individual parts or their relative assembly in order to meet specific requirements or conditions. Such changes and modifications may be made without departing from the scope of the invention, as set forth in the following claims.

## Claims

1. An apparatus for indicating fluid pressure within a fluid conduit having an outlet comprising:
(a) indicia-carrying means comprising first and second members (26, 28; 206, 208) having overlaying body portions, at least one of said members having indicating indicia viewable only upon relative sliding movement of said overlaying body portions of said members with respect to each other; and
(b) actuator means (44, 46; 224, 226) communicating with said outlet for moving said indicia-carrying means as a result of deflection thereof in response to fluid pressure being exerted on said actuator means.

2. An apparatus as defined in Claim 1 in which at least one of said members (26, 28; 206, 208) is yieldably deformable by said actuating means (44, 46; 224, 226) to cause relative movement between said members upon fluid pressure being exerted on said actuating means by fluid within the fluid conduit(16)whereby said indicating indicia becomes viewable.

3. An apparatus as defined in Claim 2, in which each said indicia-carrying member (26, 28; 206, 208) includes a body portion and an edge portion and in which said apparatus further includes a platform (20; 200) to which said edge portion of said pair of indicia carrying members (26, 28; 206, 208) are affixed, said platform having a central planar portion for supporting said thin films in an overlaying configuration wherein said body portions thereof are in close proximity.

4. An apparatus as defined in Claim 3 in which said platform (20; 200) includes an aperture (34; 214) and in which said actuator means comprises a yieldably deformable member (44; 224) aligned with said aperture, said deformable member being disposed proximate said outlet.

5. An apparatus as defined in Claim 4, further including a support plate (22; 202) disposed proximate said platform (20;200) for supporting said pair of indicia carrying members (26, 28; 206, 208), said support plate having a depression (40;222) aligned with said aperture (34; 214) in said platform, said deformable member (44; 224) being adapted to urge a portion of one (28; 208) of said thin films (26, 28;206, 208) into said depression (40; 222) upon fluid pressure being exerted on said yieldably deformable member (44; 224) of said actuating means, whereby said body portion of said one of said thin films will be caused to move relative to said body portion of said other of said thin films.

6. An apparatus as defined in Claim 5 in which said support plate (22; 202) includes a viewing aperture (22a; 202a) disposed in alignment with said body portions of said pair of thin films.

7. An apparatus according to Claim 1, adapted for visually indicating fluid pressure within first and second fluid conduits each having an outlet, wherein
(a) said first and second members respectively comprise first and second thin films (26; 28; 206, 208) having generally planar body portions disposed in an overlaying relationship, at least one of said first and second thin films having indicating indicia viewable upon relative sliding movement between said generally planar portions of said first and second films; and
(b) said actuator means (44, 46; 224, 226) communicates with said fluid outlets of said first and second fluid conduits for moving portions of said first and second films relative to each other in response to fluid pressure being exerted on said actuator means by fluid within said first and second fluid conduits.

8. An apparatus as defined in Claim 7 in which each of said first and second thin films (26, 28; 206, 208) has a fixedly located edge portion and a body portion, said first and second thin films being yieldably deformable by said actuating means to cause relative movement between said body portions of said thin films upon fluid pressure being exerted on said actuating means by fluid within said first and second fluid conduits, whereby said indicating indicia becomes viewable.

9. An apparatus as defined in Claim 8 further including:
(a) a platform (20; 200) to which said edge portions of said first and second films (26, 28; 206, 208) are affixed, said platform having spaced apart first and second apertures (34, 36; 214, 216); and
(b) a support plate (22; 202) disposed proximate said platform, said first and second films being disposed between said support plate and said platform, said support plate having spaced apart first and second depressions (40, 42; 220, 222) aligned with said apertures in said base and a viewing aperture disposed intermediate said depressions.

10. An apparatus as defined in Claim 9 in which said actuator means comprises:
(a) a first actuator element (44; 224) aligned with said first aperture (34; 214) in said platform (20; 200) for moving a portion of said first film (28; 208) into said first depression in response to fluid pressure being exerted on said first actuator element by fluid within said first fluid conduit; and
(b) a second actuator element (46; 226) aligned with said second aperture (36; 216) in said platform for moving a portion of said second film (26; 206) into said second depression in response to fluid pressure being exerted on said second actuator element by fluid within said second fluid conduit.

11. An apparatus as defined in Claim 10 in which said first and second actuator elements (44, 46; 224, 226) comprise yieldably deformable, elements overlaying said first and second apertures in said platform.

12. An apparatus according to Claim 7, comprising:
- a platform (20; 200) having first and second apertures (34, 36; 214, 216);
- a support (22; 202) connected to said platform and having a first receiving channel (40; 220) indexable with said first aperture (34; 214) in said platform and a second receiving channel (42; 222) indexable with said second aperture (36; 216) in said platform; and in that said actuator means comprises:
- a first deformable actuator element (44; 224) aligned with said first aperture (34; 214) in said platform (20; 200) and disposed between said outlet of said first conduit and said first aperture, said first actuator element being deformable from an initial configuration into an extended configuration in response to fluid under pressure within said first conduit;
- a second deformable actuator element (46; 226) aligned with said second aperture (36; 216) in said base (20; 200) and disposed between said outlet of said second conduit and said second aperture, said second actuator element being deformable from an initial configuration into an extended configuration in response to the fluid under pressure within said second conduit; and
- said first thin film (28; 208) is yieldably deformable and has a generally planar body portion and an edge portion connected to said platform (20; 200); and
- said second thin film (26; 206) is yieldably deformable and has an edge portion and a generally planar body portion partially overlaying said body portion of said first thin film, said edge portion of said second thin film being connected to said platform (20; 200) whereby said first and second thin films are disposed intermediate said platform and said support for relative sliding movement between said generally planar body portions thereof by said actuator elements between first and second positions.

13. An apparatus as defined in Claim 12 in which said support (22; 202) includes a viewing aperture (22a; 202a) for viewing a selected one of said first and second thin films.

14. An apparatus as defined in Claim 13 in which movement of said first thin film (28; 208) by said first actuator element (44; 224) permits viewing of a first indicia on said selected thin film through said viewing aperture (22a; 202a) and in which movement of said second thin film (26; 206) by said second actuator element (46; 226) permits viewing of a second indicia on said selected thin film through said viewing aperture.

15. An apparatus as defined in Claim 14 in which a third indicia is viewable through said viewing aperture when said first and second actuator elements (44, 46; 224, 226) are in said initial configuration.

16. Use of the apparatus according to Claim 1 in a device comprising:
(a) a base (132);
(b) stored energy means for forming in conjunction with said base a fluid reservoir (146) having an outlet, said stored energy means comprising a distendable member (134) superimposed over said base, said member being distendable as a result of fluid introduced into said fluid reservoir to establish internal stresses within said member tending to return said member toward a less distended configuration to force fluids from said reservoir through said outlet;
(c) a fluid conduit (152, 154, 156) connected to said outlet of said reservoir for carrying fluid forced from said reservoir by said distendable member, the apparatus for indicating pressure being operatively connected to said fluid conduit and comprising:
(d) a pair of thin films (206, 208) having edge portions and generally planar body portions disposed in an overlaying relationship defining said first and second members; and
(e) said actuator means (224, 226) communicating with said fluid conduit for engaging one of said edge portions of said thin films to move at least one of said thin films relative to the other in response to fluid pressure being extended on said actuator means by fluid carried by said fluid conduit.

17. Use as defined in Claim 16, wherein said device further includes flow control means disposed between said outlet of said fluid reservoir (146) and said pair of thin films (206, 208) for controlling the rate of fluid flow from said outlet toward said pair of thin films.

18. Use as defined in Claim 16 in which each of said pair of thin films (206, 208) has a fixedly located edge portion and a body portion, at least one of said films being yieldably deformable by said actuating means (224, 226) to cause relative movement between said body portions of said thin films upon fluid pressure being exerted on said actuating means by fluid within said fluid conduit, whereby said indicating indicia becomes viewable.

19. Use as defined in Claim 18, wherein the apparatus for indicating fluid pressure further includes:
(a) a platform (200) to which said edge portions of said first and second films are affixed, said platform having spaced apart first and second apertures (214, 216); and
(b) a support place (202) disposed proximate said platform, said first and second films being disposed between said support plate and said platform, said support plate having spaced-apart first and second depressions (220, 222) aligned with said apertures in said base and a viewing aperture disposed intermediate said depression.

20. Use as defined in Claim 19 in which said actuator means comprises:
(a) a first actuator element (224) aligned with said first aperture (214) in said platform for moving a portion of said first film (208) into said first depression (222) in response to fluid pressure being exerted on said first actuator element by fluid within said first fluid conduits; and
(b) a second actuator element (226) aligned with said second aperture (216) in said platform for moving a portion of said second film (206) into said second depression (220) in response to fluid pressure being exerted on said second actuator element by fluid within said second fluid conduit.

## Patentansprüche

1. Gerät zur Anzeige des Fluiddruckes innerhalb einer Fluidleitung mit einem Ausgang,
umfassend:
(a) Symbolträgermittel, die erste und zweite Elemente (26, 28; 206, 208) mit über einander liegenden Körperabschnitten umfassen, wobei mindestens eines dieser Elemente Anzeigesymbole besitzt, die nur bei einer relativen Verschiebung zu einander der über einander liegenden Körperabschnitte der Elemente sichtbar sind;
und
(b) Betätigungsmittel (44, 46; 224, 226), die mit dem Ausgang in Verbindung stehen, um die Symbolträgermittel als Ergebnis ihrer Biegung im Ansprechen auf den auf die Betätigungsmittel ausgeübten Druck zu bewegen.

2. Gerät nach Anspruch 1, bei dem mindestens eines der Elemente (26, 28; 206, 208) durch die Betätigungsmittel (44, 46; 224, 226) nachgiebig verformbar ist, um die relative Verschiebung zwischen den Elementen zu betragen, sobald auf die Betätigungsmittel seitens des Fluids innerhalb der Fluidleitung (16) ein Fluiddruck ausgeübt wird, wodurch die Anzeigesymbole sichtbar werden.

3. Gerät nach Anspruch 2, bei dem jedes der Symbolträgerelemente (26, 28; 206, 208) einen Körperabschnitt und einen Randabschnitt umfasst und bei dem das Gerät mindestens eine Plattform (20; 200) umfasst, an welcher der Randabschnitt des Paars von Symbolträgerelementen (26, 28; 206, 208) befestigt ist, wobei die Plattform einen ebenen, mittigen Abschnitt zur Abstützung von dünnen Filmen in einer überlappenden Konfiguration besitzt, bei der die Körperabschnitte eng aneinander liegen.

4. Gerät nach Anspruch 3, bei dem die Plattform (20; 200) eine Öffnung (34; 214) umfasst und bei dem die Betätigungsmittel ein nachgiebiges Element (44; 224) umfassen, das mit der Öffnung ausgerichtet ist, wobei das nachgiebige Element in der Nähe der Öffnung angeordnet ist.

5. Gerät nach Anspruch 4, umfassend überdies eine Tragplatte (22; 202), die in der Nähe der Plattform (20; 200) zur Abstützung des Paares von Symbolträgermittel (26, 28; 206, 208) angeordnet ist, wobei die Tragplatte eine Vertiefung (40; 222) besitzt, die mit der Öffnung (34; 214) in der Plattform ausgerichtet ist, wobei das nachgiebige Element (44; 224) einen Abschnitt eines (28; 208) der dünnen Filme (26, 28; 206, 208) in die Vertiefung (40; 222) schiebt, sobald Fluiddruck auf das nachgiebige Element (44; 224) der Betätigungsmittel ausgeübt wird, wodurch der Körperabschnitt eines der dünnen Filme gegenüber dem Körperabschnitt des anderen der dünnen Filme verstellt wird.

6. Gerät nach Anspruch 5, bei dem die Tragplatte (22; 202) eine Sichtöffnung (22a; 202a) umfasst, die mit den Körperabschnitten des Paars von dünnen Filmen ausgerichtet ist.

7. Gerät nach Anspruch 1, das den Druck des Fluids innerhalb von ersten und zweiten Fluidleitungen sichtbar anzeigt, wobei jede einen Austritt besitzt, bei dem:
(a) die ersten und zweiten Elemente jeweils erste und zweite dünne Filme (26, 28; 206, 208) umfassen, die im wesentlichen ebene, über einander liegende Körperabschnitte besitzen, wobei mindestens einer der ersten und zweiten dünnen Filme Anzeigesymbole besitzt, die bei der relativen Verschiebung zwischen den im wesentlichen ebenen Abschnitten der ersten und zweiten, dünnen Filme sichtbar werden; und
(b) Betätigungsmittel (44, 46; 224, 226) mit den Fluidausgängen der ersten und zweiten Fluidleitungen in Verbindung stehen, um Abschnitte der ersten und zweiten Filme im Ansprechen auf den Fluiddruck zu einander zu verschieben, der vom Fluid innerhalb der ersten und zweiten Fluidleitungen ausgeübt wird.

8. Gerät nach Anspruch 7, bei dem jeder der ersten und zweiten, dünnen Filme (26, 28; 206, 208) einen fest angeordneten Randabschnitt und eine Körperabschnitt besitzt, wobei die ersten und zweiten, dünnen Filme durch die Betätigungsmittel nachgiebig verformbar sind, um die relative Verschiebung zwischen den Körperabschnitten der dünnen Filme zu betragen, sobald auf die Betätigungsmittel seitens des Fluids innerhalb der ersten und zweiten Fluidleitungen ausgeübt wird, wodurch die Anzeigesymbole sichtbar werden.

9. Gerät nach Anspruch 8, weiterhin umfassend:
(a) eine Plattform (20; 200), an der die ersten Randabschnitte der ersten und zweiten, dünnen Filme (26, 28; 206, 208) befestigt sind, wobei die Plattform erste und zweite beabstandete Öffnungen (34, 36; 214, 216) besitzt; und
(b) eine Tragplatte (22; 202), die in der Nähe der Plattform angeordnet ist, wobei die ersten und zweiten Filme zwischen der Tragplatte und der Plattform angeordnet sind, wobei die Tragplatte erste und zweite, beabstandete Vertiefungen (40, 42; 220, 222), die mit den Öffnungen in der Basis ausgerichtet sind, und eine Sichtöffnung besitzt, die zwischen den Vertiefungen angeordnet ist.

10. Gerät nach Anspruch 9, bei dem die Betätigungsmittel umfassen:
(a) ein erstes Betätigungselement (44;224), das mit der ersten Öffnung (34; 214) in der Plattform (20; 200) ausgerichtet ist, um einen Abschnitt des ersten Films (28; 208) in die erste Vertiefung im Ansprechen auf den Fluiddruck zu verschieben, der auf das erste Betätigungselement durch das Fluid innerhalb der ersten Fluidleitung ausgeübt wird; und
(b) ein zweites Betätigungselement (46; 226), das mit der zweiten Öffnung (36; 216) in der Plattform ausgerichtet ist, um einen Abschnitt des zweiten Films (26; 206) in die zweite Vertiefung im Ansprechen auf den Fluiddruck zu verschieben, welcher der auf das zweite Betätigungselement durch das Fluid innerhalb der zweiten Fluidleitung ausgeübt wird.

11. Gerät nach Anspruch 10, bei dem die ersten und zweiten Betätigungselemente (44, 46; 224, 226) nachgiebige Elemente umfassen, die über den ersten und zweiten Öffnungen in der Plattform angeordnet sind.

12. Gerät nach Anspruch 7, umfassend:
- eine Plattform (20, 200) mit ersten und zweiten Öffnungen (34, 36; 214, 216);
- einen Halter (22; 202), der mit der Plattform verbunden ist und einen ersten Aufnahmekanal (40, 220), der in Position mit der ersten Öffnung (34; 214) in der Plattform einstellbar ist, und einen zweiten Aufnahmekanal (42; 222) besitzt, der in Position mit der zweiten Öffnung (36; 216) in der Plattform einstellbar ist; und wobei die Betätigungsmittel umfassen:
- ein erstes verformbares Betätigungselement (44; 224), das mit der ersten Öffnung (34; 214) in der Plattform (20; 200) ausgerichtet und zwischen dem Austritt der ersten Leitung und der ersten Öffnung angeordnet ist, wobei das erste Betätigungselement von einer Ausgangskonfiguration in eine ausgedehnte Konfiguration im Ansprechen auf das Druckfluid innerhalb der ersten Leitung verformbar ist;
- ein zweites verformbares Betätigungselement (46; 226), das mit der zweiten Öffnung (36; 216) in der Basis (20; 200) ausgerichtet und zwischen dem Ausgang der zweiten Leitung und der zweiten Öffnung angeordnet ist, wobei das zweite Betätigungselement von einer Ausgangskonfiguration in eine vergrößerte Konfiguration in Ansprechen auf das Druckfluid innerhalb der zweiten Leitung verformbar ist; und bei dem
- der erste dünne Film (28;206) nachgiebig ist und einen Randabschnitt und einen im wesentliche ebenen Körperabschnitt besitzt, der mit der Plattform (20; 200) verbunden ist; und
- der zweite dünne Film (26; 206) nachgiebig ist und einen Randabschnitt und eine im wesentlichen ebenen Körperabschnitt besitzt, der den Körperabschnitt des ersten dünnen Films überlappt, wobei der Randabschnitt des zweiten dünnen Films mit der Plattform (20; 200) verbunden ist, wodurch die ersten und zweiten dünnen Filme zwischen der Plattform und dem Halter zur Verschiebung zwischen ihren im wesentlichen ebenen Körperabschnitten seitens der Betätigungselemente zwischen den ersten und zweiten Positionen angeordnet sind.

13. Gerät nach Anspruch 12, bei dem der Halter (22, 202) eine Sichtöffnung (22a; 202) umfasst, um eine Auswahl von ersten und zweiten dünnen Filmen sichtbar zu machen.

14. Gerät nach Anspruch 13, bei dem die Verschiebung des ersten dünnen Films (28; 208) über das erste Betätigungselement (44; 224) die Sichtbarmachung eines ersten Symbols auf dem dünnen, ausgewählten Film durch die Sichtöffnung (22a; 202a) und bei dem die Verschiebung des zweiten dünnen Films (26; 206) über das zweite Betätigungselement (46; 226) die Sichtbarmachung eines zweiten Symbols auf dem ausgewählten dünnen Film durch die Sichtöffnung erlaubt.

15. Gerät nach Anspruch 14, bei dem ein drittes Symbol durch die Sichtöffnung sichtbar wird, sobald die ersten und zweiten Betätigungselemente (44, 46; 224, 226) sich in der Ausgangskonfiguration befinden.

16. Verwendung eines Gerätes nach Anspruch 1 in einer Vorrichtung umfassend:
(a) eine Basis (132);
(b) Energiespeichermittel um zusammen mit der Basis einen Fluidspeicher (146) mit einem Ausgang zu bilden, wobei die Energiespeichermittel ein über der Basis liegendes ausdehnbares Organ (134) umfassen, wobei das Organ als Ergebnis des in den Fluidspeicher eingeführten Fluids dehnbar ist, um die Innenbeanspruchungen im Organ festzustellen, die dazu neigen das Organ zu einer weniger gedehnten Konfiguration zurückzuführen um die Fluide durch die Öffnung aus dem Speicher zu drücken;
(c) eine Fluidleitung (152, 154, 156), die mit dem Ausgang des Speichers verbunden ist, um das Fluid, das seitens des dehnbaren Organs aus dem Speicher gedrückt wurde, zu fördern, wobei die Vorrichtung zur Anzeige des Druckes wirksam mit der Fluidleitung verbunden ist, und umfassend:
(d) ein Paar von dünnen Filmen (206, 208) mit Randabschnitten und im wesentlichen ebenen Körperabschnitten, die über einander liegen und die ersten und zweiten Elemente festlegen; und
(e) die Betätigungsmittel (24, 226), die mit der Fluidleitung verbunden sind, um einen der Randabschnitte der dünnen Filme zu ergreifen um mindestens einen der dünnen Filme gegenüber dem anderen im Ansprechen zum Fluiddruck zu verschieben, der auf die Betätigungsmittel durch das durch die Fluidleitung geförderte Fluid ausgedehnt wird.

17. Verwendung nach Anspruch 16, bei der die Vorrichtung überdies Fluidsteuermittel umfasst, die zwischen dem Ausgang des Fluidspeichers (146) und dem Paar von dünnen Filmen (206, 208) angeordnet sind, um den Fluiddurchfluß vom Ausgang zum Paar von dünnen Filmen zu steuern.

18. Verwendung nach Anspruch 16, bei der jedes Paar von dünnen Filmen (206, 208) einen fest positionierten Randabschnitt und einen Körperabschnitt besitzt, wobei mindestens einer der Filme seitens der Betätigungsmittel (224, 226) nachgiebig verformbar ist, um die Verschiebung zwischen den Körperabschnitten der dünnen Filme zu betragen, sobald Fluiddruk auf die Betätigungsmittel durch das Fluid innerhalb der Fluidleitung ausgeübt wird, wodurch das Anzeigesymbol sichtbar wird.

19. Verwendung nach Anspruch 18, bei der das Gerät zur Anzeige des Fluiddrucks überdies umfasst:
(a) eine Plattform (200), an der die Randabschnitte der ersten und zweiten Filme befestigt sind, wobei die Plattform erste und zweie beabstandete Öffnungen (214, 216) aufweist; und
(b) eine Tragplatte (202), die in der Nähe der Plattform angeordnet ist, wobei die ersten und zweiten Filme zwischen der Tragplatte und der Plattform angeordnet sind, wobei die Tragplatte erste und zweite beabstandete Vertiefungen (220, 222), die mit den Öffnungen in der Basis ausgerichtet sind, und eine Sichtöffnung aufweist, die zwischen den Vertiefungen angeordnet ist.

20. Verwendung nach Anspruch 19, bei der die Betätigungsmittel umfassen:
(a) ein erstes Betätigungselement (224), das mit der ersten Öffnung (214) in der Plattform ausgerichtet ist, um einen Abschnitt des ersten Films (208) in die erste Vertiefung (222) im Ansprechen zum Fluiddruck zu verschieben, der auf das erste Betätigungselement durch das Fluid innerhalb der ersten Fluidleitungen ausgeübt wird; und
(b) ein zweites Betätigungselement (226), das mit der zweiten Öffnung (216) in der Plattform ausgerichtet ist, um einen Abschnitt des zweiten Films (206) in der zweiten Vertiefung (220) im Ansprechen zum Fluiddruck zu verschieben, der auf das zweite Betätigungselement durch das Fluid innerhalb der zweiten Fluidleitung ausgeübt wird.

## Revendications

1. Appareil pour indiquer la pression d'un fluide à l'intérieur d'un conduit de fluide ayant une sortie, comprenant:
(a) des moyens porteurs de symboles comprenant des premiers et deuxièmes éléments (26, 28; 206, 208) ayant des portions de corps superposées, au moins l'un desdits éléments ayant des symboles indicateurs qui ne peuvent être visualisés que lors du mouvement de coulissement relatif desdites portions de corps superposées desdits éléments l'une par rapport à l'autre; et
(b) des moyens actionneurs (44, 46; 224, 226) communiquant avec ladite sortie pour déplacer lesdits moyens porteurs de symboles en conséquence de leur flexion en réponse à la pression de fluide exercée sur lesdits moyens actionneurs.

2. Appareil selon la revendication 1, dans lequel au moins l'un desdits éléments (26, 28; 206, 208) est déformable d'une façon souple par lesdits moyens actionneurs (44, 46; 224, 226) en vue de causer le mouvement relatif entre lesdits éléments quand une pression de fluide est exercée sur lesdits moyens actionneurs par le fluide à l'intérieur du conduit de fluide (16), de telle sorte que lesdits symboles indicateurs deviennent susceptibles de visualisation.

3. Appareil selon la revendication 2, dans lequel chacun desdits éléments porteurs de symboles (26, 28; 206, 208) comporte une portion de corps et une portion de bord et dans lequel ledit appareil comporte en outre une plate-forme (20; 200) à laquelle est fixée ladite portion de bord de ladite paire d'éléments porteurs de symboles (26, 28; 206, 208), ladite plate-forme ayant une portion centrale plate pour soutenir lesdites pellicules minces selon une configuration de superposition, où lesdites portions de corps de ces dernières sont étroitement voisines.

4. Appareil selon la revendication 3, dans lequel ladite plate-forme (20; 200) comporte une ouverture (34; 214) et dans lequel lesdits moyens actionneurs comportent un élément déformable d'une façon souple (44; 224) aligné à ladite ouverture, ledit élément déformable étant disposé proche de ladite sortie.

5. Appareil selon la revendication 4, comprenant en outre une plaque support (22; 202) disposée proche de ladite plate-forme (20; 200) pour soutenir ladite paire d'éléments porteurs de symboles (26, 28; 206, 208), ladite plaque support ayant un creux (40; 222) aligné à ladite ouverture (34; 214) dans ladite plate-forme, ledit élément déformable (44; 224) étant destiné à pousser une portion de l'une (28; 208) desdites pellicules minces (26, 28; 206, 208) dans ledit creux (40; 222) quand une pression de fluide est exercée sur ledit élément déformable d'une façon souple (44; 224) desdits moyens actionneurs, de telle sorte que ladite portion de corps de ladite une desdites pellicules minces sera amenée à se déplacer par rapport à ladite portion de corps de l'autre desdites pellicules minces.

6. Appareil selon la revendication 5, dans lequel ladite plaque support (22; 202) comporte une ouverture de visualisation (22a; 202a) disposée en alignement avec lesdites portions de corps de ladite paire de pellicules minces.

7. Appareil selon la revendication 1, destiné à indiquer visuellement la pression du fluide à l'intérieur de premiers et deuxièmes conduits de fluide, chacun ayant une sortie, dans lequel:
(a) lesdits premier et deuxième éléments comportent respectivement des premières et deuxièmes pellicules minces (26, 28; 206, 208) ayant des portions de corps généralement plates disposées en superposition, au moins l'une desdites première et deuxième pellicules minces ayant des symboles indicateurs susceptibles d'être visualisés lors du mouvement de coulissement relatif entre lesdites portions généralement plates desdites première et deuxième pellicules minces; et
(b) lesdits moyens actionneurs (44, 46; 224, 226) communiquant avec lesdites sorties de fluide desdits premier et deuxième conduits de fluide pour déplacer des portions desdites première et deuxième pellicules, l'une par rapport à l'autre, en réponse à la pression de fluide qui est exercée sur lesdits moyens actionneurs par le fluide à l'intérieur desdits premier et deuxième conduits de fluide.

8. Appareil selon la revendication 7, dans lequel chacun desdites première et deuxième pellicules minces (26, 28; 206, 208) a une portion de bord mise en place de manière fixe et une portion de corps, lesdites première et deuxième pellicules minces étant déformables d'une façon souple par lesdits moyens actionneurs en vue de causer le mouvement relatif entre lesdites portions de corps desdites pellicules minces quand une pression de fluide est exercée sur lesdits moyens actionneurs par le fluide à l'intérieur desdits premier et deuxième conduits de fluide, de telle sorte que lesdits symboles indicateurs deviennent susceptibles de visualisation.

9. Appareil selon la revendication 8, comprenant en outre:
(a) une plate-forme (20; 200) à laquelle sont fixées lesdites portions de bord desdites première et deuxième pellicules minces (26, 28; 206, 208), ladite plate-forme ayant des premières et deuxièmes ouvertures espacées (34, 36; 214, 216); et
(b) une plaque support (22, 202) disposée proche de ladite plate-forme, lesdites première et deuxième pellicules étant disposées entre ladite plaque support et ladite plate-forme, ladite plaque support ayant des premiers et deuxièmes creux espacés (40, 42; 220, 222) alignés auxdites ouvertures dans ladite base et une ouverture de visualisation disposée entre lesdits creux.

10. Appareil selon la revendication 9, dans lequel lesdits moyens actionneurs comportent:
(a) un premier élément actionneur (44; 224) aligné à ladite première ouverture (34; 214) dans ladite plate-forme (20; 200) pour déplacer une portion de ladite première pellicule (28; 208) dans ledit premier creux en réponse à une pression de fluide exercée sur ledit premier élément actionneur par le fluide à l'intérieur dudit premier conduit de fluide; et
(b) un deuxième élément actionneur (46; 226) aligné à ladite deuxième ouverture (36; 216) dans ladite plate-forme pour déplacer une portion de ladite deuxième pellicule (26; 206) dans ledit deuxième creux, en réponse à une pression de fluide qui est exercée sur ledit deuxième élément actionneur par le fluide à l'intérieur dudit deuxième conduit de fluide.

11. Appareil selon la revendication 10, dans lequel lesdits premier et deuxième éléments actionneurs (44, 46; 224, 226) comportent des éléments déformables d'une façon souple superposés auxdites première et deuxième ouvertures dans ladite plate-forme.

12. Appareil selon la revendication 7, comprenant:
- une plate-forme (20; 200) ayant des premières et deuxièmes ouvertures (34, 36; 214, 216);
- un support (22; 202) relié à ladite plate-forme et ayant un premier canal de réception (40, 220) réglable en position avec ladite première ouverture (34; 214) dans ladite plate-forme et un deuxième canal de réception (42; 222) réglable en position avec ladite deuxième ouverture (36; 216) dans ladite plate-forme; et dans lequel lesdits moyens actionneurs comportent:
- un premier élément actionneur déformable (44; 224) aligné à ladite première ouverture (34; 214) dans ladite plate-forme (20; 200) et disposé entre ladite sortie dudit premier conduit et ladite première ouverture, ledit premier élément actionneur étant déformable d'une configuration initiale à une configuration étendue en réponse au fluide sous pression à l'intérieur dudit premier conduit;
- un deuxième élément actionneur déformable (46; 226) aligné à ladite deuxième ouverture (36; 216) dans ladite base (20; 200) et disposé entre ladite sortie dudit deuxième conduit et ladite deuxième ouverture, ledit deuxième élément actionneur étant déformable d'une configuration initiale à une configuration étendue en réponse au fluide sous pression à l'intérieur dudit deuxième conduit; et dans lequel
- ladite première pellicule mince (28, 208) est déformable d'une façon souple et a une portion de corps généralement plate et une portion de bord reliée à ladite plate-forme (20; 200); et
- ladite deuxième pellicule mince (26; 206) est déformable d'une façon souple et a une portion de bord et une portion de corps généralement plate qui se superpose partiellement à ladite portion de corps de ladite première pellicule mince, ladite portion de bord de ladite deuxième pellicule mince étant reliée à ladite plate-forme (20; 200), de telle sorte que lesdites première et deuxième pellicules minces sont disposées entre ladite plate-forme et ledit support pour le mouvement de coulissement relatif entre lesdites portions de corps généralement plates de celles-ci par lesdits éléments actionneurs entre des premières et deuxièmes positions.

13. Appareil selon la revendication 12, dans lequel ledit support (22; 202) comporte une ouverture de visualisation (22a, 202a) destinée à visualiser l'une sélectionnée desdites première et deuxième pellicules minces.

14. Appareil selon la revendication 13, dans lequel le mouvement de ladite première pellicule mince (28; 208) par ledit premier élément actionneur (44; 224) permet la visualisation d'un premier symbole sur ladite pellicule mince sélectionnée à travers ladite ouverture de visualisation (22a; 202a) et dans lequel le mouvement de ladite deuxième pellicule mince (26; 206) par ledit deuxième élément actionneur (46; 226) permet la visualisation d'un deuxième symbole sur ladite pellicule mince sélectionnée à travers ladite ouverture de visualisation.

15. Appareil selon la revendication 14, dans lequel on peut visualiser un troisième symbole à travers ladite ouverture de visualisation quand lesdits premier et deuxième éléments actionneurs (44, 46; 224, 226) se trouvent dans ladite configuration initiale.

16. Emploi d'un appareil selon la revendication 1 dans un dispositif comprenant:
(a) une base (132);
(b) des moyens d'énergie accumulée pour former, ensemble avec ladite base, un réservoir de fluide (146) ayant une sortie, lesdits moyens d'énergie accumulée comprenant un organe dilatable (134) superposé sur ladite base, ledit organe étant dilatable à la suite du fluide introduit dans ledit réservoir de fluide pour établir des contraintes internes dans ledit organe tendant à causer le retour dudit organe vers une configuration moins dilatée pour pousser les fluide depuis ledit réservoir à travers ladite sortie;
(c) un conduit de fluide (152, 154, 156) relié à ladite sortie dudit réservoir pour transporter le fluide poussé dudit réservoir par ledit organe dilatable, l'appareil pour indiquer la pression étant relié de manière opérationnelle audit conduit de fluide et comprenant:
(d) une paire de pellicules minces (206, 208) ayant des portions de bord et généralement des portions de corps plates disposées en superposition, définissant lesdits premier et deuxième éléments; et
(e) lesdits moyens actionneurs (224, 226) communiquant avec ledit conduit de fluide pour engager l'une desdites portions de bord desdites pellicules minces pour déplacer au moins l'une desdites pellicules minces par rapport à l'autre, en réponse à la pression de fluide qui est étendue sur lesdits moyens actionneurs par le fluide transporté par ledit conduit de fluide.

17. Emploi comme défini dans la revendication 16, où ledit dispositif comporte en outre des moyens de commande de fluide disposés entre ladite sortie dudit réservoir de fluide (146) et ladite paire de pellicules minces (206, 208) pour commander le débit de fluide depuis ladite sortie vers ladite paire de pellicules minces.

18. Emploi comme défini dans la revendication 16, où chacune de ladite paire de pellicules minces (206, 208) a une portion de bord mise en place de manière fixe et une portion de corps, au moins l'une desdites pellicules étant déformable d'une façon souple par lesdits moyens actionneurs (224, 226) en vue de causer le mouvement relatif entre lesdites portions de corps desdites pellicules minces quand une pression de fluide est exercée sur lesdits moyens actionneurs par le fluide à l'intérieur dudit conduit de fluide, de telle sorte que ledit symbole indicateur devient susceptible de visualisation.

19. Emploi comme défini dans la revendication 18, où l'appareil pour indiquer la pression de fluide comporte en outre:
(a) une plate-forme (200) à laquelle sont fixées lesdites portions de bord desdites première et deuxième pellicules, ladite plate-forme ayant des premières et deuxièmes ouvertures espacées (214, 216); et
(b) une plaque support (202) disposée proche de ladite plate-forme, lesdites première et deuxième pellicules étant disposées entre ladite plaque support et ladite plate-forme, ladite plaque support ayant des premiers et deuxièmes creux espacés (220, 222) alignés auxdites ouvertures dans ladite base et une ouverture de visualisation disposée entre lesdits creux.

20. Emploi comme défini dans la revendication 19, où lesdits moyens actionneurs comportent:
(a) un premier élément actionneur (224) aligné à ladite première ouverture (214) dans ladite plate-forme pour déplacer une portion de ladite première pellicule (208) dans ledit premier creux (222) en réponse à une pression de fluide qui est exercée sur ledit premier élément actionneur par le fluide à l'intérieur desdits premiers conduits de fluide; et
(b) un deuxième élément actionneur (226) aligné à ladite deuxième ouverture (216) dans ladite plate-forme pour déplacer une portion de ladite deuxième pellicule (206) dans ledit deuxième creux (220) en réponse à une pression de fluide qui est exercée sur ledit deuxième élément actionneur par le fluide à l'intérieur dudit deuxième conduit de fluide.
